# EUROPEAN PATENT SPECIFICATION

(11) **EP 0 395 375 B1**
(45) Date of publication and mention of the grant of the patent: **21.08.1996**
(21) Application number: 90304432.9
(22) Date of filing: 25.04.1990
(51) Int. Cl.: C12N 15/57, C12N 1/11, C12N 5/10, C12N 9/64, A61K 38/46, A61L 27/00

(54) **Novel DNA sequences which encode ancrod-like polypeptides and compositions comprising the expression products thereof**
Für Ancrod-ähnliche Polypeptide kodierende DNA-Sequenzen und deren Expressionsprodukte enthaltende Zusammensetzungen
Séquence d'ADN codant pour des polypeptides assimilables à l'ancrod et compositions comprenant les produits d'expression de celles-ci

(30) Priority: 26.04.1989 US 343782
(43) Date of publication of application: 31.10.1990
(73) Proprietor: KNOLL AG, D-67061 Ludwigshafen (DE)
(72) Inventor: Gray, John Gordon, Jr., Raleigh, NC 27607 (US); Parikh, Indu, Chapel Hill, NC 27514 (US)
(74) Representative: Karau, Wolfgang, Dr.

(56) References cited:
- EP-A- 0 323 722
- DE-A- 2 163 899
- US-A- 4 585 653
- JOURNAL OF BIOLOGICAL CHEMISTRY, vol. 262, no. 7, 05 March 1987, American Society of Biological Chemists Inc., US; N. ITOH et al., pp. 3132-3135
- JOURNAL OF BIOLOGICAL CHEMISTRY, vol. 262, no. 26, 15 September 1987, American Society for Biochemistry & Molecular Biology Inc., US; W. KISIEL et al., pp. 12607-12613

## Description

### TECHNICAL FIELD OF THE INVENTION

The present invention relates to novel DNA sequences which encode polypeptides displaying the activity of ancrod and to recombinant DNA molecules which comprise them. The invention also relates to hosts transformed with these DNA molecules as well as to the recombinant polypeptides expressed by them. This invention further relates to pharmaceutically effective compositions and methods which employ these recombinant polypeptides.

### BACKGROUND ART

It has been known for many years that the venom of certain snakes contains a component which has anticoagulant activity. One of these components, ancrod, has been isolated from the venom of the Malayan pit viper, Agkistrodon rhodostoma (United States patent 3,743,722 and United States patent 3,879,369). Ancrod is a heavily glycosylated enzyme with a molecular weight of about 38,000. Although native ancrod has been known for many years, preparations thereof are of undefined purity. Moreover, DNA sequences encoding the polypeptide have never been isolated or synthesized and its amino acid sequence has never been determined.

Normal clot formation is mediated by thrombin, which cleaves both fibrinopeptide A and fibrinopeptide B from fibrinogen to produce fibrin, the major component of blood clots. Ancrod exerts its anticoagulant effects by cleaving only fibrinopeptide A from fibrinogen. This results in a molecule which cannot be further cleaved by thrombin and which is removed from circulation by normal mechanisms. Ancrod has also been reported to accelerate the dissolution of clots which are already formed. This effect is indirect and has been attributed to ancrod's ability to induce in vivo synthesis of tissue plasminogen activator (tPA) and/or urokinase.

Ancrod has been available for clinical use throughout much of the world for the past 20 years. It has been shown to be at least as effective as conventional anticoagulants, such as heparin and warfarin, but considerably safer in terms of fewer bleeding complications [Z. S. Latallo, "Retrospective Study on Complications and Adverse Effects of Treatment with Thrombin-Like Enzymes - A Multicenter Trial", Thromb. Haemostasis, 50, pp. 604-09 (1983)]. Clinical studies have shown ancrod to be both safe and effective in preventing deep vein thrombosis, improving renal function in patients with glomerulonephritis associated with glomerular thrombi, reversing hypercoagulable states and rapid organ deterioration associated with systemic lupus erythematosus, reducing and eliminating symptoms in both unstable and stable angina pectoris and dissolving thrombi and preventing neurological deterioration in thrombotic stroke syndromes [G. D. O. Lowe et al., "Subcutaneous Ancrod in Prevention of Deep Vein Thrombosis After Operation for Fractured Neck of Femur", Lancet, 2, pp. 698-700 (1978); S. Kim et al., "Successful Treatment with Ancrod of Glomerulonephritis with Glomerular Thrombi is Due to Activation of Fibrinolysis", Clin. Res., 34, p. 698A (1986); A K. Dosekun et al., "Ancrod in Systemic Lupus Erythematosus with Thrombosis: Clinical and Fibrinolysis Effects", Arch. Intern. Med., 144, pp. 37-42 (1984); P. Glas-Greenwalt et al., "Ancrod: Normalization of Fibrinolytic Enzyme Abnormalities in Patients with Systemic Lupus Erythematosus and Lupus Nephritis", J. Lab. Clin. Med., 105, pp. 99-107 (1985); M.B. Leon et al., "Long-Term Clot-Specific Thrombolytic Therapy in Patients with Refractory Chronic Stable Angina: Angiographic, Coronary Blood Flow, and Metabolic Changes". Circulation, 70, p. 323 (1984); V. Hossman et al., "Controlled Trial of Ancrod in Ischemic Stroke", Archives of Neurology, 40 (N13), pp 803-808, 1987].

Ancrod has also been used successfully in combination with streptokinase and urokinase to enhance thrombolysis and to prevent the reocclusion phenomena commonly seen after administration of these latter agents [B. Cercek et al., "Ancrod Enhances the Thrombolytic Effect of Streptokinase and Urokinase", Thromb. Res., 47, pp. 417-26 (1987)].

Despite its effectiveness to date, the administration of Ancrod for longer than about six weeks causes patients receiving this compound to develop immune responses. It is unclear whether these antibodies are elicited to ancrod, to impurities contained in the administered compositions or to a combination of both. The resulting anti-ancrod antibodies diminish or obliterate the effects of ancrod. At present, there are only two ways to effectively continue to treat patients who develop these antibodies. One way is to administer other, less effective or more dangerous drugs. The other way is to administer an altered form of ancrod, such as neuraminidase-treated ancrod (NTA) (United States Patent 4,585,653 ). NTA lacks the sialic acid residues present on native ancrod.

Another obstacle to the widespread use of ancrod is its availability and cost. The compound is only available from its natural source, snake venom. Therefore, manufacture of commercially feasible quantities of the compound requires facilities for maintaining snakes, as well as experts to collect their venom. Furthermore, only a limited amount of venom may be collected from a snake over a given period of time.

Accordingly, the need still exists for a compound which displays the desired properties of ancrod, but which can be produced more efficiently and in commercially feasible amounts. Moreover, such a compound should also be administerable over long periods of time without eliciting an immune response which decreases its effectiveness.

### SUMMARY OF THE INVENTION

The present invention solves the problem referred to above by providing DNA sequences which code on expression for ancrod and ancrod-like polypeptides. This invention also provides recombinant DNA molecules comprising these DNA sequences. Furthermore, expression of these DNA sequences in bacterial hosts results in the production of ancrod-like polypeptides which lack the sugar moieties which are characteristic of native ancrod. This feature may be advantageous in reducing the immune response elicited in patients treated with the recombinant bacterial polypeptide of this invention as compared to treatment with the natural, glycosylated polypeptide.

This invention also provides compositions and methods for dissolving and preventing the formation of blood clots which employ the recombinant ancrod-like polypeptides of this invention. These compositions and methods are especially useful in the treatment of thrombolytic stroke and cardiac disease.

As will be appreciated from the disclosure to follow, the present invention allows for an almost limitless supply of ancrod-like polypeptides which can be produced more rapidly and less expensively than their natural counterparts. Moreover, the ancrod-like polypeptides of this invention may be surprisingly and unexpectedly safer to use and more potent as compared with the naturally occurring compound.

### BRIEF DESCRIPTION OF THE DRAWINGS

Figure 1 depicts the nucleotide sequences of the 38 oligonucleotides used in the construction of a synthetic gene encoding an ancrod-like polypeptide.

Figure 2 depicts the nucleotide sequence of a synthetic gene encoding an ancrod-like polypeptide and the location of the oligonucleotides used in its construction.

Figure 3 depicts the nucleotide sequence of a cDNA which encodes an ancrod-like polypeptide.

Figure 4 depicts the nucleotide sequence of a synthetic gene encoding an ancrod-like polypeptide.

### DETAILED DESCRIPTION OF THE INVENTION

As used in the present specification and claims the term "ancrod-like polypeptide" refers to a polypeptide which displays the fibrinogenolytic activity of native ancrod (i.e., cleaves fibrinopeptide A, but not fibrinopeptide B from fibrinogen) or the ability to induce in vivo synthesis of tPA or urokinase. These ancrod-like polypeptides are the molecules which consist of the amino acid sequence of native ancrod as well as the molecule which contains the substitutions Lys-58→His and Asn-217→Asp.

The present invention relates to DNA sequences which code on expression for the ancrod-like polypeptides. Isolation or synthesis of the DNA sequences of the invention initially requires the elucidation of all or part of the amino acid sequence of naturally occurring ancrod.

Methods of isolating ancrod are well known in the art and are the subject of several United States patents (United States patents 3,743,722 and 3,879,369). Because the isolated product will be subjected to amino acid sequencing it is most preferable that the ancrod be purified to as great a degree as possible. Therefore, the preferred purification of ancrod comprises an initial purification step utilizing affinity chromatography on agmatine-agarose, followed by the adsorbtion of contaminants onto an anion-exchange chromatography resin, preferably DEAE. The final steps of purification employ HPLC, preferably utilizing another ionexchange resin, such as DEAE, followed by a reverse-phase resin, such as phenyl.

Because ancrod is a relatively large polypeptide, the determination of its amino acid sequence requires the generation of peptide fragments which are small enough to sequence. Those of skill in the art will recognize that the entire amino acid sequence cannot be determined by sequencing the intact polypeptide.

The generation of proteolytic fragments may be achieved by any of a number of well-known techniques. Preferably, ancrod fragments will be generated by treating the polypeptide with compounds that cleave at specific amino acids. These compounds include, but are not limited to, cyanogen bromide, trypsin, chymotrypsin, endoproteinase Asp-N and endoprotease Lys-C.

It will be obvious to those of skill in the art that any intramolecular bonds which exist in native ancrod, especially disulfide bonds, must be broken prior to cleavage and sequencing. Methods for destroying intramolecular protein bonds are well known in the art. These methods typically involve the treatment of the protein with a reducing agent, such as β-mercaptoethanol or dithiothreitol, to reduce the disulfide bond, followed by alkylation of the resulting sulfhydryl moieties. Alkylation prevents the disulfide bonds from reforming and may be achieved by standard methods.

The actual sequencing of the polypeptide fragments may be achieved by conventional methods, such as Edman degradation or carboxypeptidase A digestion, followed by amino acid analysis. Most preferably, polypeptide sequencing will be carried out on an automated protein sequencer connected to an on-line amino acid analyzer. It should be noted that those amino acids which contain covalently attached sugar residues cannot be identified by the above technique. These amino acids are preferably analyzed by mass spectroscopy means.

Once the entire amino acid sequence of ancrod is determined, synthetic genes which code for all or a portion of the polypeptide backbone may be designed. These synthetic genes may also be tailored for expression in specific hosts. This may be achieved by utilizing preferential codons to code for each amino acids [M. Watson, Nucl. Acids Res., 12, pp. 5145-64 (1984)]. Alternatively, oligonucleotide probes may be designed based on the amino acid sequence of ancrod and subsequently used to screen a snake venom gland cDNA library to isolate an ancrod cDNA.

The synthesis of genes which code for ancrod-like polypeptides is carried out using standard methods known to those of skill in the art. Preferably, the genes are constructed from complementary pairs of small, sense and anti-sense synthetic oligonucleotides which encode various portions of the ancrod polypeptide sequence. It is most preferable that these complementary pairs of oligonucleotides contain 3′ or 5′ overhangs for accurate assembly.

Assembly of the oligonucleotides will initially involve the annealing of complementary oligonucleotides to one another. The annealed, complementary pairs are then ligated together via the overhangs to form an intact gene. Ligation of the oligonucleotide pairs may be performed in a single step or, more preferably, in multiple steps. Because of the size of the ancrod polypeptide it is not feasible to synthesize the gene as a single complementary pair of oligonucleotides.

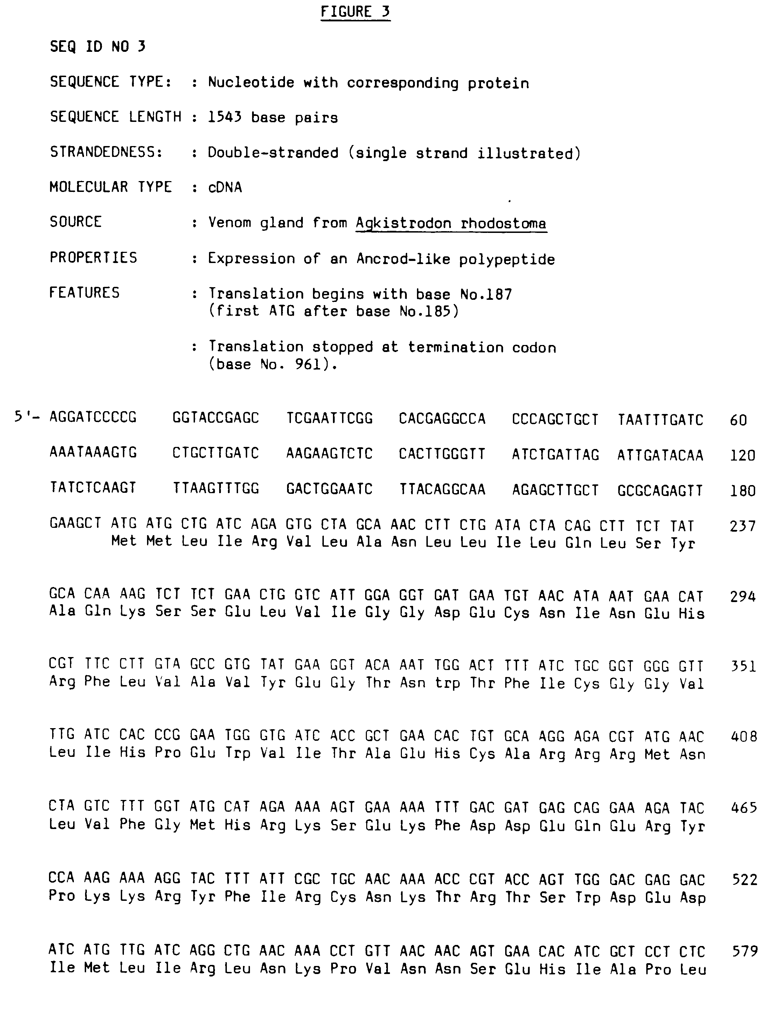

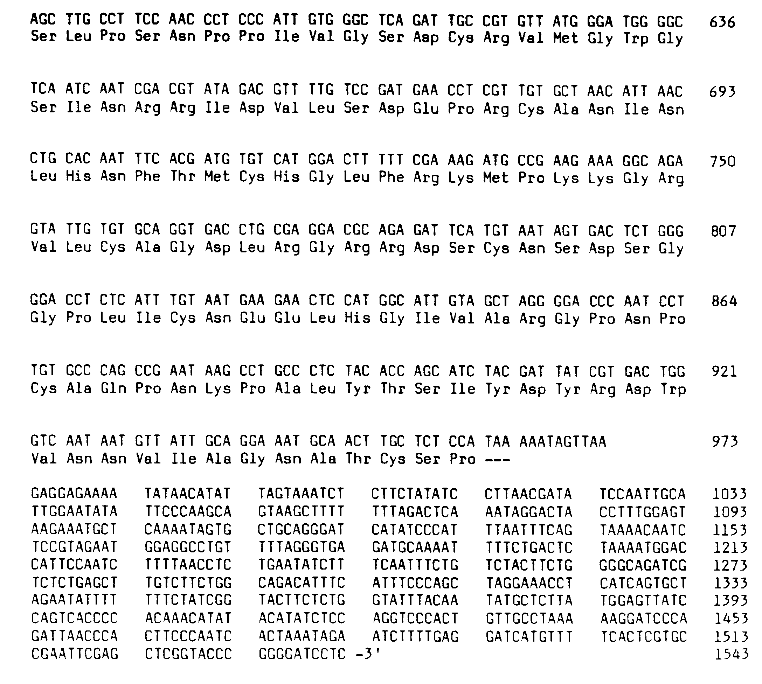

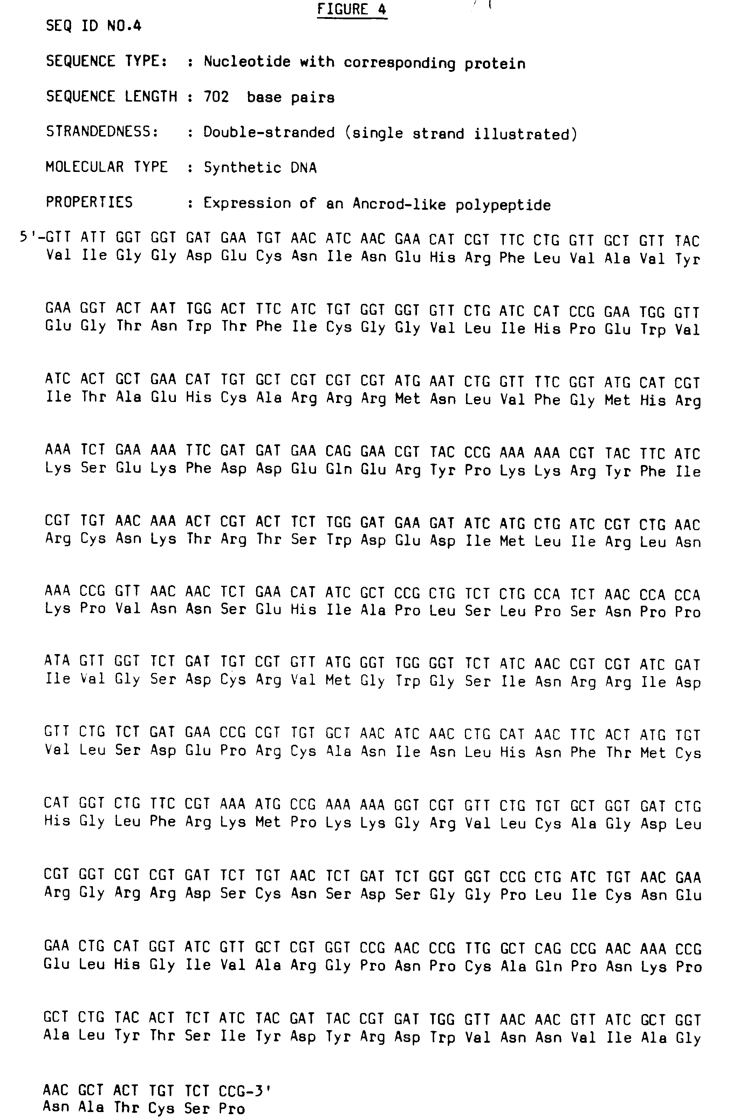

It will be understood by those of skill in the art that, due to the degeneracy of the genetic code, many different synthetic DNAs will be capable of encoding an ancrod-like polypeptide. It will also be apparent that many of these DNAs will be faithfully expressed in various hosts transformed with them. Therefore, the present invention relates to not only one, but all DNA molecules which encode the desired ancord-like polypeptide and which can be expressed by one or more hosts transformed with them. These other DNA sequences will either hybridize to the DNA sequences depicted in Figure 4 or will code on expression for the ancrod-like polypeptide coded for on expression by the DNA sequences depicted in Figure 3 or Figure 4. Hybridization conditions are exemplified by initial hybridization in 1X SSC at 42°C, followed by washing at a somewhat higher stringency, such as in 0.1 X - 1 X SSC at 58°C.

The synthetic ancrod genes of the present invention may also comprise additional nucleotides outside of the ancrod-like polypeptide coding region. Such additional nucleotides may represent various structural and functional features which allow for insertion into appropriate vectors and which optimize expression in hosts transformed with the synthetic gene. The additional nucleotides which may also comprise the synthetic ancrod gene include, but are not limited to, 5′ and 3′ restriction endonuclease sites, a 5′, in-frame secretory signal sequence, a 5′ or 3′ in-frame peptide or polypeptide such that the ancrod-like polypeptide is expressed as a fusion protein, a 3′ RNA polymerase termination sequence, a polyadenylation signal, a 5′ promoter sequence, a host-specific 5′ ribosome binding sequence (Shine-Dalgarno sequence) and an in-frame initiator methionine codon. Alternatively, any or all of these additional nucleotides may be present in an appropriate vector into which the ancrod gene may be inserted. Vectors containing these features are well known in the art.

An in-frame initiator methionine codon is required for initiation of translation useful because the synthetic ancrod genes of this invention code for a mature polypeptide which lacks an amino-terminal methionine. This feature may be supplied as a 5′, in-frame secretory sequence; a 5′, in-frame peptide or polypeptide; or a 5′, in-frame ATG.

A 5′, in-frame ATG codon will elicit direct translation of an ancord-like polypeptide in a transformed host. Those of skill in the art will recognize that the expression product of such a gene will be met-ancrod. Such a product may be modified by standard methods to remove the extra methionine residue or used directly if it possesses effective biological activity.

Initiation effected by a DNA sequence which codes for an in-frame signal sequence at the 5′ end of the gene, a structural feature which codes for an initiator methionine followed by approximately 20 amino acids, provides a targeting mechanism by which the ancrod-like polypeptide may be secreted from the host. Secretion is desirable because the ancrod-like polypeptide may be more easily purified from the growth media of hosts transformed with the recombinant DNA molecules of this invention, and, in the case of gram negative bacterial hosts, from the periplasmic space, than from the cellular millieu. And, the use of a signal sequence obviates the need for modifying the final expression product because the signal sequence should be removed in vivo by the transformed host.

Initiation effected by a 5′, in-frame DNA sequence which codes for a peptide or polypeptide will result in the expression of a fusion protein. The DNA which codes for the 5′ peptide or polypeptide may be either homologous or heterologous to the host used for expression. The DNA may already be present in the cloning vector into which the synthetic ancrod-like gene is to be or it may be ligated to the 5′ end of the synthetic ancrod-like gene prior to insertion into the vector. The DNA may be obtained from a natural source, or, more preferably, be synthesized by standard techniques.

The size of the 5′ peptide or polypeptide may range from 2 amino acids up to about 1000 amino acids. Most preferably the size of the peptide or polypeptide will range from about 2 amino acids to about 100 amino acids. The 5′ peptide or polypeptide may consist of any amino acid sequence as long as it includes the presence of a functional initiator methionine. Those of skill in the art will recognize that the amino acid sequence of the 5′ peptide or polypeptide may confer various characteristics upon the fusion protein which may or may not be desirable. These characteristics include varying degrees of solubility within the host cell and varying susceptibility to host cell degradation systems. Once purified, the expressed fusion protein may be modified by chemical and enzymatic methods well known in the art to remove the 5′ peptide or polypeptide from the ancrod-like polypeptide. Alternatively, the fusion protein may be used directly if it possesses effective biological activity.

The isolation of a cDNA coding for an ancrod-like polypeptide may be achieved by methods which are well known in the art. Initially, a snake venom gland cDNA library must be synthesized. This involves the isolation of snake venom gland mRNA, the synthesis of first and second strand DNA, the addition of appropriate linkers or adapters to the ends of the cDNA molecules and the insertion of the cDNAs into an appropriate vector. All of these techniques are standard procedures employed by those skilled in the art.

Once the cDNA library is obtained, clones containing ancrod cDNA sequences are identified by probing the library with labeled oligonucleotide probes. Such probes will be synthesized based on the ancrod polypeptide sequence. The synthesis of the oligonucleotide probes will preferably be performed using a automated oligonucleotide synthesizer. It will be obvious to those of skill in the art that the choice of oligonucleotide probes will be based upon stretches of amino acids from different parts of the ancrod molecule which are encoded by DNA sequences of low redundancy. Preferably, the oligonucleotide probes are: 5'-GTTCATTTATATTACATTCATCACCTCCAATGAC-3' and 5'-CCGTGACACATCGTGAAGTTGTG-3'. The former oligonucleotide is based on amino acids 1-12 of ancrod, while the latter seqeuence is based on amino acids 147-152. Alternatively, the synthetic ancrod-like gene described above may be used as a probe.

The probes may be labeled by any of a number of techniques which are well known to those in the art [T. Maniatis et al., A Molecular Guide to Cloning: A Laboratory Manual, Cold Spring Harbor Laboratories, New York (1982)]. Most preferably, labeling is achieved by ³²P end-labeling. The screening of the cDNA with labeled oligonucleotide probes may be carried out using well-known techniques (T. Maniatis et al., supra).

Once an ancrod cDNA clone has been identified and isolated, the cDNA insert may be removed from the vector and analyzed to determine whether it contains the entire ancrod polypeptide coding sequence. If it does not contain a full-length cDNA, the insert itself may be used to reprobe the cDNA library and to locate full-length cDNAs. The partial cDNA may also be converted to a full length cDNA by adding synthetic oligonucleotides [D. Goeddel et al., "Direct Expression in Escherichia coli of a DNA Sequence Coding for Human Growth Hormone", Nature, 281, pp. 544-48 (1979)]. Alternatively, if the partial cDNA codes for a biologically active portion of the ancrod molecule it will fall within the scope of this invention and it may be used directly to transform hosts which, in turn, will express an ancord-like polypeptide.

The above-described cDNA screening method may similarly be used to isolate genomic clones containing ancrod genes. In this case, snake venom gland genomic DNA is isolated, cleaved into appropriately sized pieces by a restriction enzyme and inserted into an appropriate vector to create a genomic library. Such techniques are well known in the art. The genomic library may then be screened by identical methods to those used for screening cDNA libraries.

The DNA sequences and recombinant DNA molecules of the present invention may be inserted into and expressed using a wide variety of vectors. For example, useful vectors may consist of segments of chromosomal, non-chromosomal and synthetic DNA sequences, such as various known derivatives of SV40 and known bacterial plasmids, e.g., plasmids from E.coli including colE1, pCR1, pBR322, pMB9 and RP4; phage DNAs, e.g., the numerous derivatives of λ phage, e.g., NM 989, and other DNA phages, e.g., M13 and other Filamentous single-stranded DNA phages; vectors useful in yeasts, such as the 2µm plasmid; vectors useful in animal cells, such as those containing SV40, adenovirus and retrovirus-derived DNA sequences; and vectors derived from combinations of plasmids and phage DNAs, such as plasmids which have been modified to employ phage DNA or other derivatives thereof.

Such expression vectors are also characterized by at least one expression control sequence that may be operatively linked to the ancrod-like polypeptide DNA sequence inserted in the vector in order to control and to regulate the expression of that cloned DNA sequence. Examples of useful expression control sequences include the lac system (e.g. lac UV5), the trp system, the tac system, the trc system, the major operator and promoter regions of phage λ (P_{L} or P_{R}), the control region of fd coat protein, the bacteriophage T₇ promoter, the glycolytic promoters of yeast, e.g., the promoter for 3-phosphoglycerate kinase, the promoters of yeast acid phosphatase, e.g., Pho5, the promoters of the yeast-mating factors, and promoters derived from polyoma, adenovirus, retrovirus, and simian virus, e.g., the early and late promoters of SV40, and other sequences known to control the expression of genes of prokaryotic or eukaryotic cells and their viruses or combinations thereof. Preferred expression control sequences are tac, trp, trc, lacUV5, P_{L}, P_{R}, and T₇. Most preferred is P_{R}.

Among such useful expression vectors are vectors that enable the expression of the cloned polypeptide in eukaryotic hosts, such as animal and human cells [e.g., P. J. Southern and P. Berg, J. Mol. Appl. Genet., 1, pp. 327-41 (1982); S. Subramani et al., Mol. Cell. Biol., 1, pp. 854-64 (1981); R. J. Kaufmann and P. A. Sharp, "Amplification And Expression Of Sequences Cotransfected with A Modular Dihydrofolate Reductase Complementary DNA Gene", J. Mol. Biol., 159, pp. 601-21 (1982); R. J. Kaufmann and P. A. Sharp, Mol. Cell. Biol., 159, pp. 601-64 (1982); S. I. Scahill et al., "Expression And Characterization Of The Product Of A Human Immune Interferon DNA Gene In Chinese Hamster Ovary Cells", Proc. Natl. Acad. Sci. USA, 80, pp. 4654-59 (1983); G. Urlaub and L. A. Chasin, Proc. Natl. Acad. Sci. USA, 77, pp. 4216-20 (1980)].

Furthermore, within each specific expression vector, various sites may be selected for insertion of DNA sequences encoding an ancrod-like polypeptide. These sites are usually designated by the restriction endonuclease which cuts them. They are well recognized by those of skill in the art. It is, of course, to be understood that an expression vector useful in this invention need not have a restriction endonuclease site for insertion of the chosen DNA fragment. Instead, the vector may be joined to the fragment by alternative means.

The expression vector, and in particular the site chosen therein for insertion of a selected DNA fragment and its operative linking therein to an expression control sequence, is determined by a variety of factors, e.g., number of sites susceptible to a particular restriction enzyme, size of the protein to expressed, susceptibility of the desired protein to proteolytic degradation by host cell enzymes, contamination or binding of the protein to be expressed by host cell proteins difficult to remove during purification, expression characteristics, such as the location of start and stop codons relative to the vector sequences, and other factors recognized by those of skill in the art. The choice of a vector and an insertion site for a DNA sequence is determined by a balance of these factors, not all selections being equally effective for a given case.

Useful hosts which may be transformed with these vectors and which may be employed to express the polypeptides of this invention may include well known eukaryotic and prokaryotic hosts, such as strains of E.coli, such as E.coli XL-1, E.coli SG-936, E.coli HB 101, E.coli W3110, E.coli X1776, E.coli X2282, E.coli DHI, and E.coli MRCl, Pseudomonas, Bacillus, such as Bacillus subtilis and Streptomyces.

Of course, not all host/expression vector combinations will function with equal efficiency in expressing the DNA sequences of this invention or in producing the ancrod-like polypeptide. However, a particular selection of a host-expression vector combination may be made by those of skill in the art, after due consideration of the principles set forth herein without departing from the scope of this invention. For example, the selection should be based on a balancing of a number of factors. These include, for example, compatibility of the host and vector, toxicity of the proteins encoded by the DNA sequence to the host, ease of recovery of the desired protein, expression characteristics of the DNA sequences and the expression control sequences operatively linked to them, biosafety, costs and the folding, form or any other necessary post-expression modifications of the desired protein. Preferred hosts are strains of E.coli. Most preferred are derivatives of pKK233-2.

It will be obvious to those of skill in the art that the expression of the full-length ancrod-like DNA sequences of this invention will produce a polypeptide that differs from native ancrod in that it lacks glycosylation.

Confirmation of the expression of an ancrod-like polypeptide may be achieved by any of a large number of assays which are well known in the art. These assays may be based upon the detection of ancrod-specific structural determinants or biological activity. For example, assays of the former group include ELISA, Western Blot, immunoprecipitation and immunodiffusion techniques. These techniques all employ monoclonal or polyclonal antibodies raised against intact ancrod or fragments thereof. Biological activity assays may be either be direct (i.e., enzymatic activity) or indirect (i.e., assay of induced compounds). Direct assays include those which detect esterase activity or cleavage of fibrinopeptide A from fibrinogen. Indirect assays are exemplified by determination of in vivo induction of tPA synthesis or in vivo induction of urokinase synthesis following administration of the expression product to a patient [see for example, T. Soszcka et al., "Effect of Fibrinogen-Clotting Enzymes on Secretion of Plasminogen Activators from Cultured Human Endothelial Cells", Fibrinolysis, 2, pp. 49-57 (1988)].

The expression products of hosts transformed with the DNA sequences and recombinant DNA molecules of the present invention may be utilized in pharmaceutically effective compositions and methods for preventing or treating thromboses. The compositions of the present invention are useful in dissolving existing blood clots and in preventing the formation of new blood clots in a patient. According to a preferred embodiment, these compositions are especially useful in the prevention of deep-vein thrombosis after major surgery, in the treatment of glomerulonephritis associated with glomerular thrombi, in the reversal of hypercoagulable states and rapid organ deterioration associated with systemic lupus erythematosus, in preventing coagulation wihtout complications during cardiac surgical procedures such as cardiopulmonary bypass, in the treatment of both stable and unstable ischemic heart disease syndromes, in the prevention of the reocclusion phenomenom associated with the prior administration of urokinase or streptokinase and in the enhancement of thrombolysis and prevention of neurological deterioration in thrombotic stroke victims.

The pharmaceutically acceptable compositions of the present invention comprise a pharmaceutically effective amount of an ancrod-like polypeptide. Such amounts are between about 0.001 and about 1000 units/kg body weight of the patient to which the composition is to be administered. More preferrably, the compositions comprise between about 0.05 and about 20 units/kg body weight.

The compositions of the present invention may also comprise natural tissue plasminogen activator, recombinant tissue plasminogen activator, anisoylated plasminogen streptokinase activator complex (APSAC), streptokinase, urokinase or a combination thereof. These compositions are especially effective in treating unstable ischemic heart disease syndromes, such as unstable angina, coronary intermediate syndrome and acute myocardial infarction.

These compositions will contain pharmaceutically effective amounts of the additional components that are equal to or less than the amounts currently used in monotherapies. For example, in the compositions of the present invention, tPA is preferably used in a dosage range of about 0.1 - 50 mg/kg body weight of the patient; streptokinase is preferably used in a dosage range of about 1,000 - 1,000,000 units/kg body weight; ASPAC is preferably used in a dosage range of about .01 - 10 units/kg body weight; and urokinase is preferably used in a dosage range of about 1,000 - 1,000,000 units/kg body weight. Those of skill in the art will recognize that tPA, streptokinase and urokinase cannot be administered to a patient in a bolus injection. Therefore, the compositions of the present invention that comprise these particular compounds must be administered to a patient by intravenous means over a period of time.

The pharmaceutically acceptable compositions of the present invention preferably include at least one pharmaceutically acceptable carrier. In addition, the pharmaceutically acceptable compositions of the present invention may also comprise a pharmaceutically acceptable buffer of neutral pH, preferably phosphate buffered saline, together with a pharmaceutically acceptable compound for adjusting isotonic pressure, such as sodium chloride, mannitol or sorbitol.

Such compositions are suitably adapted for oral, parenteral and topical administration, parenteral compositions being preferred and intravenous compositions being most preferred. Compositions formulated for topical administration may, for example, be in aqueous jelly, oily suspension or emulsified ointment form. For parenteral administration, fluid unit dose forms may be prepared containing a composition or combination of the present invention and a sterile vehicle. The polypeptides contained in the pharmaceutically acceptable composition may be either suspended or dissolved, preferably dissolved, depending on the nature of the vehicle employed. Parenteral compositions are normally prepared by dissolving the polypeptides, optionally together with other components, in a vehicle and filter sterilizing before filling into a suitable vial or ampule and sealing. Preferably, adjuvants such as a local anesthetic, preservatives and buffering agents may also be dissolved in the vehicle. The composition may then be frozen and lyophilized to enhance its stability.

Parenteral suspensions are prepared in substantially the same manner, except that the polypeptides are suspended, rather than dissolved in the vehicle. Advantageously, a pharmaceutically acceptable surfactant or wetting agent is included in the composition to facilitate uniform distribution of the polypeptide and any other optional components.

Tablets and capsules for oral administration may contain conventional excipients such as binding agents, fillers, diluents, tabletting agents, lubricants, disintegrants, colorants, flavorings and wetting agents. The tablet may be coated according to methods well known in the art.

Suitable fillers for use include cellulose, mannitol, lactose and other similar agents. Suitable disintegrants include, but are not limited to, starch, polyvinylpyrrolidone and starch derivatives such as sodium starch glycollate. Suitable lubricants include, for example, magnesium stearate. Suitable wetting agents that are useful include sodium lauryl sulfate.

Oral liquid preparations may be in the form of aqueous or oily suspensions, solutions, emulsions, syrups or elixirs, or may be presented as a dry product for reconstitution with water or other suitable vehicle before use. Such liquid preparations may contain conventional additives. These include suspending agents, such as sorbitol, syrup, methyl cellulose, gelatin, hydroxyethylcellulose, carboxymethylcellulose, aluminum stearate gel or hydrogenated edible fats; emulsifying agents which include lecithin, sorbitan monooleate or acacia; non-aqueous vehicles, such as almond oil, fractionated coconut oil, and oily esters; and preservatives, such as methyl or propyl p-hydroxybenzoate or sorbic acid.

According to an alternative embodiment the recombinant ancrod-like polypeptides of the present invention may be employed in compositions and methods for coating the surface of an invasive device, resulting in the lower risk of blood clot formation in patient receiving such a device. Surfaces that may be coated according to the methods and compositions of the present invention are exemplified by those of prostheses, artificial valves, vascular grafts, stent and catheters. Methods for adhering these compositions to the surfaces of an invasive device are known in the art. These include chemical cross-linking or physical adsorption of the ancrod-like polypeptide compositions to the surfaces of these devices.

In order that this invention may be more fully understood, the following examples are set forth. It should be understood that these examples are for illustrative purposes only and are not to be construed as limiting this invention in any manner.

### EXAMPLE 1

### Purification Of Ancrod

We purified ancrod by initially dissolving 220 mg of lyophillized venom of Agkistrodon rhodostoma (Quality Venoms for Medical Research, Lot AR1552; Punta Gorda, Florida) in 8 ml of 50 mM Tris-HCl, pH 8.0, containing 30 mM NaCl (loading buffer). The solution was then loaded onto an 8 ml Agmatine-Sepharose column which was prepared according to C. Nolan et al., "Ancrod, The Coagulating Enzyme from Malayan Pit Viper (Agkistrodon rhodostoma) Venom", Methods In Enzymol., XLV, pp. 205-13 (1976). This resin binds ancrod under these initial buffer conditions. We washed the column at room temperature with 160 ml of loading buffer and then eluted the ancrod with the same buffer containing 150 mM guanidine hydrochloride. We assayed fractions (4 ml) for esterase activity by incubating 40 µl of a fraction with 1 ml of 50 mM Tris-HCl, pH 8, 1 mM Benzoyl-arginine ethylester (BAEE; Sigma, B-4500) at room temperature and monitoring the change in absorbance at 253 nm. A change of .038 OD₂₅₃/ minute equals 1 µg of ancrod.

We pooled the active fractions and applied them directly to a 5 ml DEAE-Sephacel column (Pharmacia) which had previously been equilibrated in 50 mM Tris-HCl, pH 8. The ancrod did not bind to this anion exchange resin under these conditions. Flow-through fractions were assayed for activity as described above. The active fractions (8 ml total) were pooled and subsequently desalted using an Amicon ultrafiltration cell, fitted with a YM-10 membrane (Amicon Corp., Danvers, Massachusetts). We diluted the ultrafiltrate to 8 ml with 10 mM Tris-HCl, pH 7.5 and then applied it to a 7.5 mm x 75 mm TSK DEAE 5PW HPLC column (Beckman, Berkley, California). We eluted the ancrod with a linear NaCl gradient (0.0 - 1.0 M) in 10 mM Tris-HCl, pH 7.5 over 20 minutes. The fractions were assayed for activity as described above. The pooled active fractions, which corresponded to more than 1% by weight of the starting material, was checked for purity by electrophoresis in an SDS-polyacrylamide gel followed by silver staining. We further purified ancrod on a reverse phase Brownlee Aquapore Phenyl column (2.1 x 100 mm; Applied Biosystems, Foster City, California) using a linear 0 - 100% acteonitrile gradient in 0.1% trifluoroacetic acid over 30 minutes.

### EXAMPLE 2

### Protein Sequencing Of Ancrod

We reduced the reverse-phase HPLC-purified ancrod by incubating it in 6 M guanidine-HCl, 1 M Tris-HCl, pH 8.6, 10 mM EDTA ard 20 mM DTT at 37°C for one hour at room temperature. We alkylated the resulting reduced ancrod by adding 4-vinylpyridine to a final concentration of 50 mM and incubating the solution for 30 minutes at room temperature. The alkylated polypeptide was desalted on a reverse phase Brownlee Aquapore Phenyl column (2.1 x 100 mm; Applied Biosystems) using a linear 0 - 100% acetonitrile gradient in 0.1% trifluoroacetic acid (TFA) over 30 minutes.

We carried out three different types of digestions on the alkylated ancrod to create fragments for protein sequencing. Digestion with endoprotease Asp-N (Boehringer-Mannheim, Indianapolis, Indiana) was performed in 0.1 M Tris-HCl, pH 8.5 using an enzyme-polypeptide ratio of 1:100. The digestion was allowed to proceed for 15 hours at room temperature. The resulting proteolytic fragments were separated on an Aquapore Phenyl column using a linear 0 - 40% acetonitrile gradient in 0.1% TFA over 60 minutes.

We carried out endoproteinase Lys-C (Boehringer-Mannheim, Indianapolis, Indiana) digestion in 0.1 M Tris-HCl, pH 8.5 using an enzyme-polypeptide ratio of 1:100. The digestion was performed for 40 hours at room temperature. We separated the resulting fragments on a Hypersil ODS HPLC column (2.1 x 150 mm; Hewlett-Packard, Avondale, Pennsylvania) using a linear 0 - 60% acetonitrile gradient in 0.1% TFA over 90 minutes.

We performed CNBr cleavage of intact, alkylated ancrod by incubating 12 µg of ancrod in 10% TFA containing 25 pg of CNBr. The sample was incubated for 23 hours in the dark at room temperature under a N₂ atmosphere. The resultant peptide mixture was lyophilized and redissolved in 6 M guanidine-HCl. The CNBr fragments were then separated on a Hypersil ODS HPLC column using a linear 0 - 80% acetonitrile gradient in 0.1% TFA over 60 minutes.

Each of the peptides resulting from the three digests was sequenced using an Applied Biosystems 477A liquid-phase sequencer (Applied Biosystems). Phenylthiohydantoin (PTH) amino acids were identified on-line using an Applied Biosystems 120A PTH Analyzer. The amino acid sequences were confirmed by tandem mass spectroscopy which elucidated the identification of glycosylated amino acid residues.

By sequencing the above peptide fragments of ancrod we determined the entire sequence of the polypeptide to be: 
VIGGDECNIN EHRFLVAVYE GTNWTFICGG VLIHPEWVIT
AEHCARRRMN LVFGMHRHSE KFDDEQERYP KKRYFIRCNK
TRTSWDEDIM LIRLNKPVNN SEHIAPLSLP SNPPIVGSDC
RVMGWGSINR RIDVLSDEPR CANINLHNFT MCHGLFRKMP
KKGRVLCAGD LRGRRDSCNS DSGGPLICNE ELHGIVARGP
NPCAQPNKPA LYTSIYNYRD WVNNVIAGNA TCSP.
 In the above polypeptide sequence and in the claims the amino acids are represented by the standard single letter codes as follows:

Phe: F    Leu: L    Ile: I    Met: M    Val: V
Ser: S    Pro: P    Thr: T    Ala: A    Tyr: Y
His: H    Gln: Q    Asn: N    Lys: K    Asp: D
Glu: E    Cys: C    Trp: W    Arg: R    Gly: G

The asparagine residues at amino acids 78, 99, 148 and 229 are putative N-glycosylation sites. We have not, however, determined the exact nature and numbers of the sugar residues attached to these sites.

### EXAMPLE 3

### Construction Of A Synthetic Gene Encoding An Ancrod-Like Polypeptide

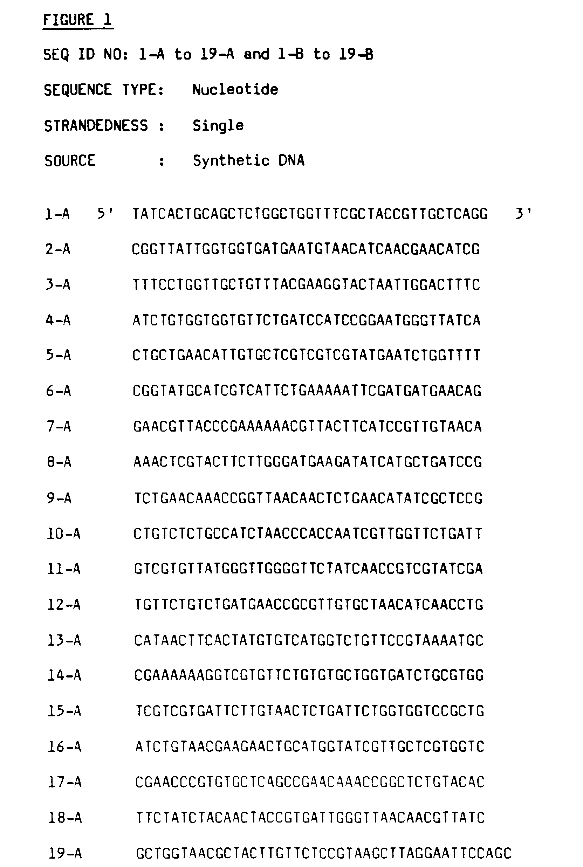

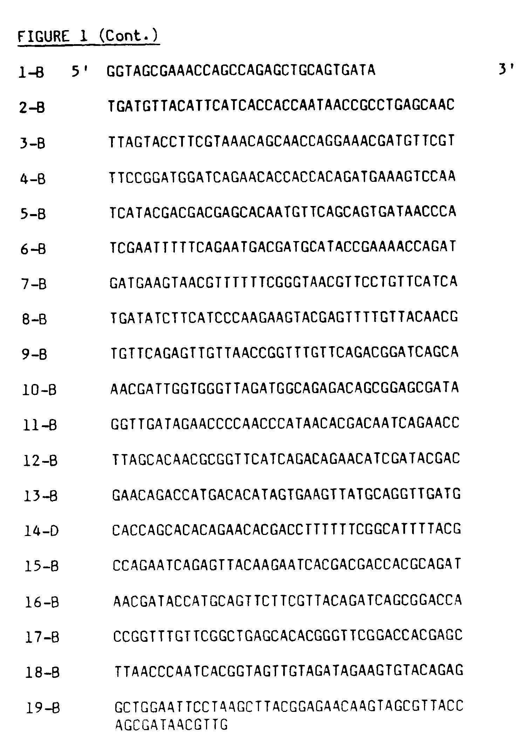

Once we had determined the amino acid sequence of ancrod, we designed and synthesized an ancrod gene for optimal expression in E.coli. To construct this gene we synthesized a series of 39 oligonucleotides (Figure 1). The synthetic ancrod gene utilized codons preferred by E.coli and contained an in-phase ATG at the 5' end of the coding region. The gene also contained EcoRI sites at the 3' and 5' ends and an internal HindIII site at the 3' end to aid in cloning the gene into an appropriate expression vector.

Specifically, the gene was synthesized as follows: For each region of the synthetic gene an oligonucleotide (1A - 19A; Figure 1) and its matching complementary oligonucleotide (1B - 19B; Figure 1) were separately synthesized. All of the oligonucleotides were 40 bases long, except for 1A (41 bases), 19A (43 bases), 1B (31 bases) and 19B (53 bases). Each set of oligonucleotides was designed to anneal over a length of 30 bases with a 10 base overhang on either end. Each member of a complementary pair of oligonucleoitdes was synthesized, annealed to its complement and phosphorylated. Complementary sets of oligonucleotides were ligated in groups as follows; 1A & B through 5A & B, 6A & B through 10A & B, 11A & B through 14A & B, 15A & B through 19A & B. We separated and purified the resulting fragments by electrophoresis in a 6% nondenaturing polyacrylamide gel [T. Maniatis, supra]. The fragments in the gel were visualized under ultraviolet light after staining with ethidium bromide. We removed gel slices containing the fragments by excision with a scalpel. The gel slices were each transferred to a 5 ml snap cap tube and crushed with a sterile glass rod. The DNA fragments were eluted by suspending the crushed gel in 1 ml of 0.5 M ammonium acetate at room temperature under constant agitation. The acrylamide was then removed by centrifugation and the resulting solution passed through a pasteur pipette stuffed with glass wool.

We brought the volume of the filtrate back up to 1 ml by adding 0.5 M ammonium acetate and subsequently concentrated the volume down to 0.2 ml by extraction with 1 N butanol. The aqueous phase, which contained the DNA fragments, was ethanol precipitated two times and finally resuspended in 10 mM Tris, 1 mM EDTA, pH 8.1 (TE buffer).

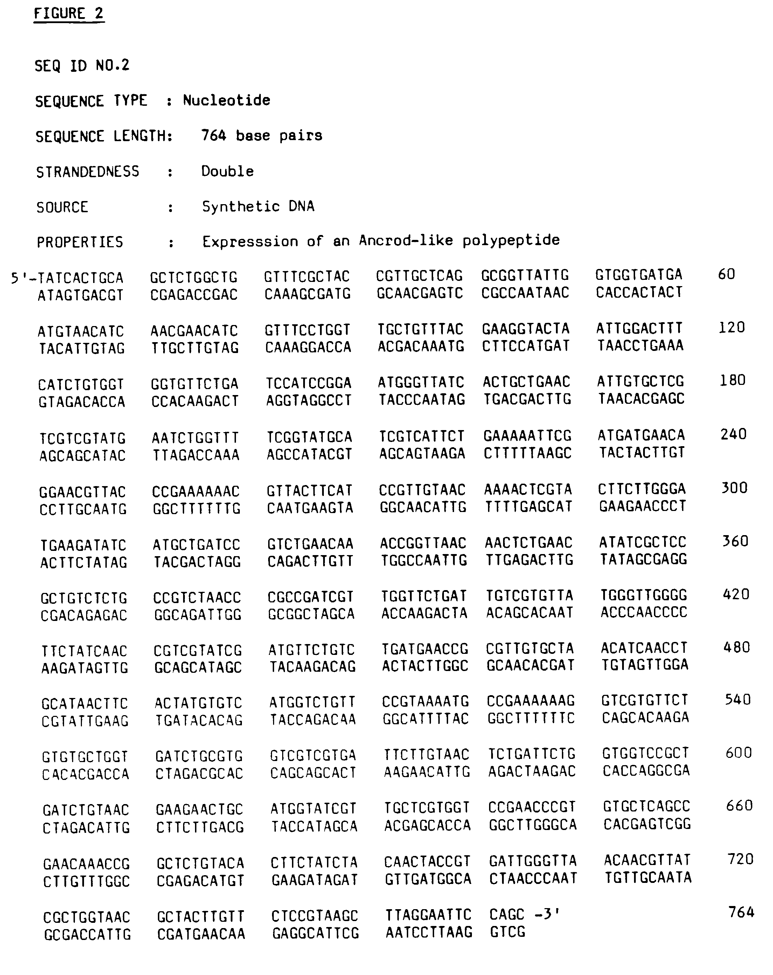

The four separately purified fragments were then ligated together and digested with EcoRI and PstI. (All restriction enzymes were purchased from either Bethesda Research Laboratories or New England Biolabs. All digestions were carried out according to manufacturer's directions.) We purified the resulting 754 base pair fragment using a nondenaturing polyacrylamide gel as described above. The purified fragment was then ligated into EcoRI/PstI cut pUC18 (Bethesda Research Latoratories, Gaithersburg, Maryland). We next removed the fragment from pUC18 by EcoRI digestion and purified it by electrophoresis in low-melt agarose (Bethesda Research Laboratories). The purified EcoRI fragment was then ligated into EcoRI digested M13 mp19 for DNA sequencing. The sequence of the synthetic gene was determined by dideoxy chain termination sequencing [F. Sanger et al., "DNA Sequencing with Chain-Terminating Inhibitors", Proc. Natl. Acad. Sci. USA, 74, pp. 5463-67 (1977)]. The sequence of the final construct (Figure 2) differed from the predicted sequence at positions 330, 339, 342 and 345, but these base changes were degenerate and did not change the encoded amino acid sequence. We believe these mutations occurred during the subcloning process.

### EXAMPLE 4

### Isolation Of A cDNA Clone Containing An Ancrod-Like DNA Sequence

A venom gland (Ventoxin Labs, Inc., Frederick, Maryland) from Agkistrodon rhodostoma was frozen in liquid nitrogen and placed in a mortar which was kept chilled in a dry ice-ethanol bath. The gland was chipped into small pieces with a scalpel and poly A+ RNA was isolated from the disrupted tissue using a "Fast Track mRNA isolation kit" (Invitrogen, San Diego, California) according to manufacturer's instructions.

We then synthesized double-stranded cDNA from the isolated mRNA using a "cDNA kit" (Pharmacia LKB Biotechnology, Inc., Piscataway, New Jersey) according to manufacturer's instructions. We ligated aliquots of the final cDNA preparation, which contained EcoRI ends, into EcoRI-digested and dephosphorylated lambda gt11 arms (Promega; Madison, Wisconsin). The ligated DNA was packaged using lambda packaging extract prepared according to standard procedures [T. Maniatis et al., Molecular Cloning: A Laboratory Manual, Cold Spring Harbor Laboratory, New York (1982)].

The entire, unamplified library consisted of approximately 7.5 x 10⁵ plaque forming units (pfu). We plated 8 x 10³ pfu on each of ten 100 mm LB petri dishes. We screened the plaques with two different ³²P-end-labeled oligonucleotide probes using standard lambda library screening techniques [T. Maniatis et al., supra]. The two probes were designed based on the amino acid sequence of natural ancrod which was determined as in Example 2. The first probe, which was the reverse complement of a nucleotide sequence which coded for amino acids 1 through 12, consisted of the nucleotide sequence: 5'-GTTCATTTATATTACATTCATCACCTCCAATGAC-3' The second oligonucleotide probe was the reverse complement of a nucleotide sequence which codes for amino acids 147-154 of natural ancrod. Its nucleotide sequence was: 5'-CCGTGACACATCGTGAAGTTGTG-3' Both of these oligonucleotide probes were chosen by picking stretches of the amino acid sequence of ancrod that contained codons with the least redundancy. The first probe was based on the published amino terminal amino acid sequence of a different snake venom protease [N. Itoh et al., "Molecular Cloning and Sequence Analysis of cDNA for Batroxobin, a Thrombin-like Snake Venom Enzyme," J. Biol. Chem., 262, pp. 3132-35 (1987)] that matched the first 12 amino acids of ancrod, except for one amino acid. The identical codons for the matching amino acids were chosen and the codon for the different amino acid was picked according to the codon used most often for that amino acid in the batroxobin cDNA sequence. The second probe was designed by comfor each amino acid in the batroxobin gene and using those codons used most often for each amino acid in our probe.

The oligonucleotides were synthesized independently on an Applied Biosystems 381A Nucleotide Synthesizer (Applied Biosystems) according to manufacturer's instructions. The oligonucleotides were end labeled with ³²P by standard techniques.

The above screening procedure resulted in the identification of two clones each of which, upon isolation and restriction enzyme analysis, was shown to contain an approximately 1.5 kilobase pair EcoRI insert. The inserts from each of these positive clones were subsequently subcloned into M13mp18 sequencing vectors (Bethesda Research Laboratories). We determined the entire nucleotide sequence of one insert using the dideoxy chain termination method. The resulting sequence of this cDNA clone is shown in Figure 3.

A comparison of the putative amino acid sequences encoded by the synthetic gene and the cDNA revealed differences at amino acid 58 and 217. The synthetic gene encoded histidine and asparagine, respectively, at these positions. The cDNA encoded amino acids lysine and aspartic acid, respectively, at positions 58 and 217. The errors in the synthetic gene were most likely due to errors in determining the amino acid sequence of the native ancrod molecule.

Therefore, the correct amino acid sequence of native ancrod is:

VIGGDECNIN EHRFLVAVYE GTNWTFICGG
VLIHPEWVIT AEHCARRRMN LVFGMHRKSE
KFDDEQERYP KKRYFIRCNK TRTSWDEDIM
LIRLNKPVNN SEHIAPLSLP SNPPIVGSDC
RVMGWGSINR RIDVLSDEPR CANINLHNFT
MCHGLFRKMP KKGRVLCAGD LRGRRDSCNS
DSGGPLICNE ELHGIVARGP NPCAQPNKPA
LYTSIYDYRD WVNNVIAGNA TCSP

In order to obtain a synthetic gene which encoded a polypeptide that was identical to that encoded by the cDNA, we altered the synthetic gene at these two codons by site-directed mutagenesis [P. Carter et al., "Improved Oligonucleotide Site-Directed Mutagenesis Using M13 Vectors", Nucleic Acids Res., 13, pp. 4431-43 (1985)]. The sequence of the gene after mutagenesis and the amino acid sequence of the encoded polypeptide are given in Figure 4.

### EXAMPLE 5

### Expression of An Ancrod-Like Polypeptide

In order to express the synthetic ancrod-like gene produced as in Example 3 and modified by site-directed mutagenesis as in Example 4, we constructed expression vector pIP495. The vector was constructed by digesting plasmid pKK233-2 (Pharmacia LKB Biotechnology, Piscataway, NJ) with EcoRI and HindIII to remove the trc promoter cassette. A second vector pMLB1034 [C. Queen "A Vector that Uses Phage Signals for Efficient Synthesis of Proteins in Escherichia coli", J. Mol. App. Genet., 2,pp.1-10], was digested with EcoRI and BamHI to remove a 960 base pair fragment which contained both the lambda P_{R} promoter and the c1857 temperature sensitive repressor of the P_{R} promoter.

We next removed the synthetic ancrod gene, which had been previously inserted into pUC19 as described in Example 3, by digestion with EcoRII and HindIII. This digestion yielded a fragment which contained all, but the 5′ most 42 bases of the polypeptide coding region.

We then synthesized the following oligonucleotide linker to replace the missing 42 bases: 
5' CCATGGATCCCGTTATTGGTGGTGATGAATGTAACATCAACGA
3' GGTACCTAGGGCAATAACCACCACTACTTACATTGTAGTTGCT

ACATCGTTT 3'
TGTAGCAAATGGACC 5'
 The above oligonucleotide linker provides the 42 bases to complete the coding sequence for mature ancrod, plus an additional 9 base pairs at the 5′ end. These additional base pairs code for three additional amino acids, beginning with an initiator methionine and provide a BamHI recognition site. The linker was digested with BamHI prior to use in the four-way ligation described below.

The four DNA fragments, the EcoRI/HindIII digested pKK233-2, the 960 base pair P_{R} promoter with EcoRI and BamHI ends, the synthetic partial ancrod gene with EcoRII and HindIII ends and the BamHI-digested oligonucleotide, which contained BamHI and EcoRII ends, were assembled in a four piece ligation. We confirmed proper construction of the resulting plasmid, pIP495, by restriction mapping.

We then transformed E.coli XL-1 cells (Stratogene Cloning Systems, La Jolla, California) with pIP495, according to standard techniques. Positive transformants expressed a protein of 27,000 molecular weight which reacted positively with antibodies to native ancrod in a Western Blot assay. The expressed protein cleaves fibrinopeptide A from fibrinogen.

Microorganisms and recombinant DNA molecules according to this invention are exemplified by a culture deposited in the In Vitro International, Inc. culture collection in Linthicum, Maryland on April 20, 1989 and identified as:
IVI-10203 : E.coli XL-1 (pIP495)

The culture was assigned the accession number indicated above.

While we have hereinbefore presented a number of embodiments of this invention, it is apparent that our basic construction can be altered to provide other embodiments which utilize the processes and products of this invention. Therefore, it will be appreciated that the scope of this invention is to be defined by the claims appended hereto rather than the specific embodiments which have been presented hereinbefore by way of example.

## Claims (Claims for the following Contracting State(s): DK)

1. An unglycosylated ancrod-like polypeptide comprising the amino acid sequence

VIGGDECNIN EHRFLVAVYE GTNWTFICGG VLIHPEWVIT AEHCARRRMN
LVFGMHRKSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP

2. An unglycosylated ancrod-like polypeptide comprising the amino acid sequence

VIGGDECNIN EHRFLVAVYE GTNWTFICGG VLIHPEWVIT AEHCARRRMN
LVFGMHRHSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPNKPA LYISIYDYRD WVNNVIAGNA TCSP

3. The method of preparing an ancrod-like polypeptide according to claim 1 or 2 by expressing DNA sequences which code on expression for these polypeptides in bacteria.

4. A pharmaceutically acceptable composition for dissolving existing blood clots and for preventing the formation of new blood clots in a patient, said composition comprising a pharmaceutically effective amount of the ancrod-like polypeptide according to claim 1 or 2.

5. The pharmaceutically acceptable composition according to claim 4, further comprising a pharmaceutically effective amount of a compound selected from the group consisting of natural tissue plasminogen activator, recombinant tissue plasminogen activator, ASPAC, streptokinase and urokinase.

6. A composition for coating the surface of an invasive device to be inserted into a patient, said composition comprising the ancrod-like polypeptide according to claim 1 or 2.

7. A method for coating the surface of an invasive device to be inserted into a patient, said metod comprising the step of contacting said surface with a composition according to claim 6 prior to insertion into said patient.

## Claims (Claims for the following Contracting State(s): ES)

1. The method of preparing an unglycosylated acrod-like polypeptide comprising the amino acid sequence 
VIGGDECNIN EHRFLVAVYE GTNWTFICGG VLIHPEWVIT AEHCARRRMN
LVFGMHRKSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP or
 
VIGGDECNIN EHRFLVAVYE GTNWTFICGG VLIHPEWVIT AEHCARRRMN
LVFGMHRHSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPNKPA LYISIYDYRD WVNNVIAGNA TCSP
 by expressing DNA sequences which code on expression for these polypeptides in bacteria.

## Patentansprüche (Patentansprüche für folgende(n) Vertragsstaat(en): DK)

1. Nicht glykosyliertes Ancrod-ähnliches Polypeptid, enthaltend die Aminosäuresequenz

VIGGDECNIN EHRFLVAVYE GTNWTFICGG VLIHPEWVIT AEHCARRRMN
LVFGMHRKSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP.

2. Nicht glykosyliertes Ancrod-ähnliches Polypeptid, enthaltend die Aminosäuresequenz

VIGGDECNIN EHRFLVAVYE GTNWTFICGG VLIHPEWVIT AEHCARRRMN
LVFGMHRHSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP.

3. Verfahren zur Herstellung eines Ancrod-ähnlichen Polypeptids gemäß Anspruch 1 oder 2, bei dem man für diese Polypeptide kodierende DNA-Sequenzen in Bakterien exprimiert.

4. Pharmazeutisch verträgliche Zusammensetzung zur Auflösung bestehender Blutgerinnsel und zur Verhinderung der Neubildung von Blutgerinnseln, die eine pharmazeutisch wirksame Menge des Ancrod-ähnlichen Polypeptids gemäß Anspruch 1 oder 2 enthält.

5. Pharmazeutisch verträgliche Zusammensetzung nach Anspruch 4, die weiter eine pharmazeutisch wirksame Menge einer aus der Gruppe, bestehend aus natürlichem Gewebe-Plasminogen-Aktivator, rekombinant gewonnenem Gewebe-Plasminogen-Aktivator, anisoyliertem Plasminogen-Streptokinase-Aktivatorkomplex (APSAC), Streptokinase und Urokinase enthält.

6. Zusammensetzung zur Beschichtung der Oberfläche einer in den Körper eines Patienten einzuführenden Vorrichtung, die das Ancrod-ähnliche Polypeptid gemäß Anspruch 1 oder 2 enthält.

7. Verfahren zur Beschichtung der Oberfläche einer in den Körper eines Patienten einzuführenden Vorrichtung, bei dem man die Oberfläche vor dem Einführen mit einer Zusammensetzung gemäß Anspruch 6 in Berührung bringt.

## Patentansprüche (Patentansprüche für folgende(n) Vertragsstaat(en): ES)

1. Verfahren zur Herstellung eines nicht glykosylierten Ancrod-ähnlichen Polypeptids, enthaltend die Aminosäuresequenz 
VIGGDECNIN EHRFLVAVYE GTNWTFICGG VLIHPEWVIT AEHCARRRMN
LVFGMHRKSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP bzw.
VIGGDECNIN EHRFLVAVYE GTNWTFICGG VLIHPEWVIT AEHCARRRMN
LVFGMHRHSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP
 bei dem man für diese Polypeptide kodierende DNA-Sequenzen in Bakterien exprimiert.

## Revendications (Revendications pour l'(les) Etat(s) contractant(s) suivant(s): DK)

1. Un polypeptide analogue à l'ancrod, non glycosylé, comprenant la séquence d'acides aminés

VIGGDECNIN EHRFLVAVYE GTNWTFICGG VLIHPEWVIT AEHCARRRMN
LVFGMHRKSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP

2. Un polypeptide analogue à l'ancrod, non glycosylé, comprenant la séquence d'acides aminés

VIGGDECNIN EHRFLVAVYE GTNWTFICGG VLIHPEWVIT AEHCARRRMN
LVFGMHRHSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPNKPA LYISIYDYRD WVNNVIAGNA TCSP

3. Le procédé de préparation d'un polypeptide analogue à l'ancrod selon la revendication 1 ou 2, par expression de séquences d'ADN qui codent pour l'expression de ces polypeptides dans des bactéries.

4. Une composition pharmaceutiquement acceptable pour dissoudre des caillots sanguins existants et pour empêcher la formation de nouveaux caillots sanguins chez un patient, ladite composition comprenant une quantité pharmaceutiquement efficace du polypeptide analogue à l'ancrod selon la revendication 1 ou 2.

5. La composition pharmaceutiquement acceptable selon la revendication 4, comprenant, de plus, une quantité pharmaceutiquement efficace d'un composé choisi dans le groupe formé par l'activateur tissulaire du plasminogène naturel, un activateur tissulaire du plasminogène recombinant, l'ASPAC, la streptokinase et l'urokinase.

6. Une composition destinée à enduire la surface d'un dispositif effractif à introduire dans un patient, ladite composition comprenant le polypeptide analogue à l'ancrod selon la revendication 1 ou 2.

7. Un procédé pour enduire la surface d'un dispositif effractif à introduire dans un patient, ledit procédé comprenant l'étape de mise en contact de ladite surface avec une composition selon la revendication 6 avant l'introduction dans ledit patient.

## Revendications (Revendications pour l'(les) Etat(s) contractant(s) suivant(s): ES)

1. Le procédé de préparation d'un polypeptide analogue à l'ancrod, non glycosylé, comprenant la séquence d'acides aminés 
VIGGDECNIN EHRFLVAVYE GTNWTFICGG VLIHPEWVIT AEHCARRRMN
LVFGMHRKSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPDKPA LYISIYDYRD WVNNVIAGNA TCSP Ou
 
VIGGDECNIN EHRFLVAVYE GTNWTFICGG VLIHPEWVIT AEHCARRRMN
LVFGMHRHSE KFDDEQERYP KKRYFIRCNK TRTSWDEDIM LIRLNKPVNN
SEHIAPLSLP SNPPIVGSDC RVMGWGSINR RIDVLSDEPR CANINLHNFT
MCHGLFRKMP KKGRVLCAGD LRGRRDSCNS DSGGPLICNE ELHGIVARGP
NPCAQPNKPA LYISIYDYRD WVNNVIAGNA TCSP
 par expression de séquences d'ADN qui codent pour l'expression de ces polypeptides dans des bactéries.
